# FASCICULE DE BREVET EUROPEEN

(11) **EP 4 041 220 B1**
(45) Date de publication et mention de la délivrance du brevet: **26.02.2025**
(21) Numéro de dépôt: 20796637.5
(22) Date de dépôt: 06.10.2020
(51) Int. Cl.: A61K 31/336, A61P 25/28, A61K 36/02, A61K 35/748

(54) **COMPOSITION RENFERMANT DES CAROTÉNOÏDES ET LEUR UTILISATION POUR LA PROTECTION DES NEURONES CONTRE LA NEURODÉGÉNÉRATION**
CAROTINOIDE ENTHALTENDE ZUSAMMENSETZUNG UND IHRE VERWENDUNG ZUM SCHUTZ VON NEURONEN GEGEN NEURODEGENERATION
COMPOSITION CONTAINING CAROTENOIDS AND USE THEREOF FOR PROTECTING NEURONS AGAINST NEURODEGENERATION

(30) Priorité: 09.10.2019 FR 1911184
(43) Date de publication de la demande: 17.08.2022
(73) Titulaire: Serive, Benoît, 44200 Nantes (FR)
(72) Inventeur: Serive, Benoît, 44200 Nantes (FR)
(74) Mandataire: Fidal Innovation
(86) Numéro de dépôt international: PCT/FR2020/051750
(87) Numéro de publication internationale: WO 2021/069828

(56) Documents cités:
- K.S. CHO ET AL.: "Recent advances in studies on the therapeutic potential of dietary carotenoids in neurodegenerative diseases", OXIDATIVE MEDICINE AND CELLULAR LONGEVITY, vol. 2018, 16 April 2018 (2018-04-16), XP002799273
- SOONTORNCHAIBOON ET AL.: "ANTI-INFLAMMATORY EFFECTS OF VIOLAXANTHIN ISOLATED FROM MICROALGA CHLORELLA ELLIPSOIDEA IN RAW 264.7 MACROPHAGES", BIOL. PHARM. BULL., vol. 35, no. 7, 2012, pages 1137 - 1144, XP002799272
- LATOWSKI D ET AL: "Violaxanthin and diadinoxanthin cycles as an important photoprotective mechanism in photosynthesis", RUSSIAN JOURNAL OF PLANT PHYSIOLOGY, NAUKA/INTERPERIODICA, MO, vol. 58, no. 6, 12 October 2011 (2011-10-12), pages 952 - 964, XP019963398, ISSN: 1608-3407, DOI: 10.1134/S1021443711060124

## Description

La présente invention concerne le domaine des compositions renfermant des caroténoïdes, plus particulièrement de la famille des xanthophylles, et leur utilisation dans la prévention ou le traitement du déclin cognitif ou de maladies neurodégénératives.

Les caroténoïdes sont des pigments naturels présents chez différents organismes tels que les animaux, les plantes, les algues et les microorganismes. A ce jour, plus de 800 caroténoïdes sont clairement identifiés. Chez les végétaux, ces molécules leur permettent de collecter la lumière pour réaliser la photosynthèse et d'autres leur permettent une photo-protection pour dissiper l'énergie qui n'est pas directement utilisée.

Parmi les caroténoïdes, on distingue les carotènes et les xanthophylles. Les carotènes tels que le beta-carotène ou le lycopène sont des caroténoïdes hydrocarbonés stricts sans aucun substituant dans leur structure. Les xanthophylles (ou oxycaroténoïdes) qui appartiennent au second groupe sont des molécules contenant des atomes d'oxygène.

Des études passées ont pu montrer depuis un certain nombre d'années que l'alpha-carotène, le beta-carotène, la crocétine, la crocine, la beta-cryptoxanthine, la lutéine, le lycopène, la zéaxanthine, l'astaxanthine et la fucoxanthine présentaient un potentiel pour préserver les neurones de la neurodégénération. Ces molécules possèdent notamment des activités anti-oxydantes, anti-inflammatoires, et modulatrices de l'autophagie, une mort cellulaire nécessaire à l'élimination des protéines mal repliées ou des agrégats protéiques que l'on retrouve dans la plupart des maladies neurodégénératives.

Dans la publication *"*Anti-inflammatory Effects of Violaxanthin Isolated from Microalga Chlorella ellipsoidea in RAW 264. 7 Macrophages" de Waesarat Soontornchaiboon, Seong Soo Joo et Sang Moo Kim, l'effet anti-inflammatoire de la violaxanthine isolée de C. ellipsoidea a été examiné. Des études épidémiologiques sur l'homme ont validé les résultats obtenus en culture cellulaire et sur animaux concernant les bénéfices des caroténoïdes pour diminuer le risque de maladies neurodégénératives (K.S. Cho et al, Recent advances in studies on the therapeutic potential of dietary carotenoids in neurodegenerative diseases. Oxidative medicine and cellular Longevity, vol. 2018, article ID4120458).

L'invention est définie dans les revendications annexées. Dans la présente description et les dessins, tous les exemples et descriptions techniques d'appareils, de produits et/ou de méthodes qui ne sont pas couverts par les revendications doivent être considérés comme de l'art antérieur ou des exemples utiles à la compréhension de l'invention. Au cours de ses recherches et études des différents pigments caroténoïdes issus de micro-algues l'inventeur a découvert de manière surprenante que des molécules particulières de la famille des xanthophylles présentaient des effets supérieurs sur la protection de la mort cellulaire.

A cet effet la présente invention concerne une composition renfermant des pigments caroténoïdes de la famille des xanthophylles, caractérisée en ce que lesdits pigments de la famille des xanthophylles comprennent au moins l'une des molécules de formule (I) ou (II) suivantes : 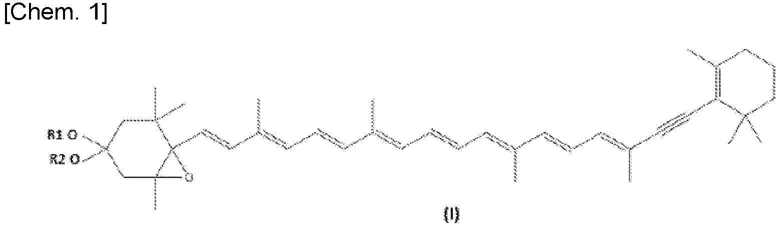 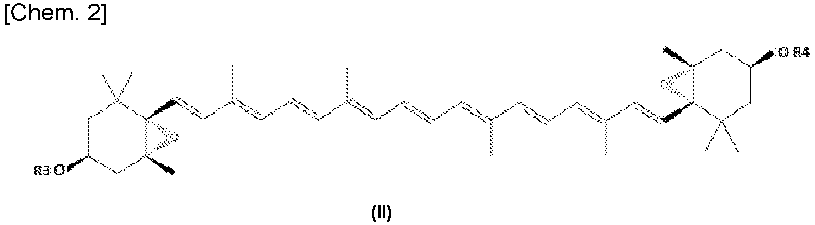 dans lesquelles les radicaux R1, R2, R3 et R4, identiques ou différents, sont choisis parmi l'hydrogène, un radical hydrocarboné linéaire ou ramifié, saturé ou insaturé, tel qu'un radical alkyle en C₁ à C₃₆, ou les groupements -OR1, -OR2, - OR3 ou -OR4 sont des esters d'acides gras en C₄ à C₃₆, de préférence en C4 à C₂₈, pour une utilisation dans la prévention ou le traitement du déclin cognitif ou de maladies neurodégénératives.

Selon un aspect particulier de l'invention, la molécule de la famille des xanthophylles de formule (I) est la diadinoxanthine, ou un ester d'acide gras en C₄ à C₃₆ de la diadinoxanthine.

Selon un autre aspect particulier de l'invention, la molécule de la famille des xanthophylles de formule (II) est la violaxanthine, ou un ester d'acide gras en C₄ à C₃₆ de la violaxanthine.

De préférence les esters d'acides gras sont des esters en C₄ à C₂₈, de préférence encore des esters en C₈ à C₁₈.

Lesdites molécules xanthophylles peuvent être extraites de micro-algues marines ou d'eau douce, de macro-algues marines, de cyanobactéries ou de bactéries. Elles peuvent aussi être issues de synthèse organique ou d'hémisynthèse.

Les molécules xanthophylles extraites des micro-algues marines ou d'eau douce sont préférées.

De manière avantageuse, les molécules xanthophylles de formule (I) sont extraites d'algues euglénophytes de l'embranchement Euglenozoa, de diatomées de l'embranchement Bacillariophyta, de dinoflagellés de la classe Dinophyceae, d'Haptophytes, de Pelagophytes, de Phaetothamniophytes, de Dictyochophytes ou d'Ochrophytes.

De manière avantageuse, les molécules xanthophylles de formule (II) sont extraites d'algues des embranchements des Chrysophytes, des Eustigmatophytes, des Synurophytes, des Mesostigmatophytes, des Chlorophytes, des Prasinophytes, des Chlorarachniophytes, des Pinguiophytes, des Raphidophytes, ou extraites des dinoflagellés, ou de macro-algues.

Dans le cas où lesdites molécules xanthophylles d'intérêts selon l'invention sont produites par des micro-organismes, celles-ci peuvent être obtenues par tout procédé adapté, tel qu'un procédé comprenant les étapes suivantes :
- Production en (photo)-bioréacteurs, en bassins ou cuves ;
- Concentration par centrifugation ou filtration ;
- Extraction solide/liquide comme par exemple assistée par microondes, par broyage à billes, par homogénéisateur, par utilisation de fluides supercritiques, par champs électrique pulsé, ou par enzymes ;
- Eventuellement, séparation/pressage ;
- Filtration comme par exemple ultrafiltration tangentielle ;
- Purification comme par exemple par chromatographie de partage centrifuge ou par chromatographie liquide haute performance ;
- Evaporation et séchage, puis conditionnement.

Préalablement au conditionnement peut être prévue l'incorporation d'additifs et d'autres ingrédients tels que des excipients, émulsifiants, agents de charge, épaississants, antiagglomérants, stabilisants, acidifiants, arômes et autres principes actifs, ainsi que l'homogénéisation de ces formulations.

Les molécules xanthophylles de formules (I) ou (II) de la composition selon l'invention peuvent être obtenues à partir de micro-algues, au moyen par exemple d'un procédé (tel que décrit dans la publication de B. SERIVE et al., PLoS ONE 12(2) :e0171872, 2017), comprenant les étapes successives suivantes :
i) broyage des micro-algues et dispersion dans de l'éthanol dans un broyeur-mélangeur à billes permettant l'extraction des xanthophylles,
ii) après élimination desdites billes, centrifugation pour recueillir le surnageant,
iii) filtration du surnageant sur un filtre de 0,2 µm,
iv) suivie immédiatement d'une séparation et purification des pigments par chromatographie de partage centrifuge ou par chromatographie liquide haute performance et détection UV des fractions éluées,
v) séchage et conditionnement.

Selon un premier mode de réalisation de l'invention, ladite composition peut être utilisée en tant que complément alimentaire ou nutritionnel.

Par complément nutritionnel on entend ici tout ingrédient dans le domaine de la nutrition santé ou toute composition utilisée en nutrition clinique spécialisée.

Le complément alimentaire peut se présenter sous forme d'un prémix, utilisé pour la préparation d'un apport dans l'alimentation humaine.

Selon un second mode de réalisation de l'invention, ladite composition peut être utilisée en tant que substance active dans un médicament.

La composition selon l'invention vise en particulier à retarder l'apparition des maladies neurodégénératives, à limiter leurs effets ou à empêcher l'apparition de celles-ci ou de leurs symptômes. On entend ici par le terme maladie neuro-dégénérative, l'ensemble des maladies où le phénomène de mort cellulaire des neurones et/ou cellules gliales de façon pathologique est impliqué. La composition selon l'invention peut ainsi être notamment utilisée dans la prévention ou le traitement de la maladie d'Alzheimer, la maladie de Parkinson, l'épilepsie, les déficiences intellectuelles/autisme, l'ataxie spinocérébelleuse, la sclérose en plaques, les démences à corps de Lewy, la maladie d'Alexander, la maladie d'Alpers, l'atrophie multi-systématisée, l'atrophie cortico postérieure (syndrome de Benson), la dégénérescence cortico-basale, la paralysie supranucléaire progressive, la maladie de Pick, la myofasciite à macrophages, la maladie de Huntington, la sclérose latérale amyotrophique, la maladie de Creutzfeldt-Jakob, ou la neurodégénération glaucomateuse telle que le glaucome primaire à angle ouvert.

La formulation est aussi particulièrement adaptée à un mode préventif lié au déclin cognitif, c'est à dire le vieillissement cellulaire aboutissant à la mort des neurones.

Ladite composition peut comprendre au moins un élément supplémentaire choisi parmi :
- les vitamines suivantes : E, B3, B5, B6, H, B9 et B12 ;
- les oligo-éléments et minéraux suivants : magnésium, iode et sélénium ;
- les composés suivants : acide eicosapentaénoïque (EPA), acide docosahexaénoïque (DHA), et lécithine de tournesol ;
- des protéines végétales ;
- un extrait de myrtille ou d'une autre plante ;
- les caroténoïdes suivants : astaxanthine, fucoxanthine, lutéine, zéaxanthine, alpha-carotène, beta-carotène, crocétine, crocine, beta-cryptoxanthine et lycopène ;
- des excipients, émulsifiants, agents de charge, épaississants, antiagglomérants, stabilisants, acidifiants et arômes.

Par ailleurs, de par la nature des molécules de la composition selon l'invention et de par les propriétés anti-oxydantes et anti-inflammatoire des ingrédients de la composition, les bénéfices peuvent être étendus aussi à l'utilisation, la prévention ou le traitement de la dégénérescence maculaire liée à l'âge, les maladies liées à la mort des ostéoclastes telle que l'ostéoporose, à la perte de poids, de l'obésité ou de l'hyperlipidémie, à la prévention du syndrome d'ischémie/reperfusion lors de la transplantation d'organes (dont le rein), à la prévention de cancers tels que celui de la prostate, du sein, du foie, des intestins, de la vessie, de la gorge, de la bouche, du mélanome, cancer de la peau, à la prévention ou au traitement des mycoses, à la prévention ou le traitement de la schizophrénie, à la prévention ou le traitement des maladies de peau, à la prévention ou au traitement des maladies inflammatoires, à la prévention ou au traitement du diabète de type 2, à la prévention ou au traitement de la stéato-hépatite non alcoolique (NASH).

La composition et les ingrédients qui la composent peuvent être également utilisés en dermato-cosmétique pour améliorer la beauté en protégeant la peau contre les UV A et B, en tant qu'anti-rides, en tant qu'agent anti-tyrosinase, pour prévenir la formation de radicaux libres oxydants qui causent le vieillissement cellulaire de la peau.

La composition selon l'invention peut se présenter par exemple, de manière non exhaustive, sous la forme de gélules, sous forme de capsules, sous forme de stick liquide, sous forme de liquide, sous forme de comprimé, sous forme de gel, sous forme de poudre, sous forme d'ampoules buvables, sous forme injectable, sous forme de compte-gouttes.

### Brève description des dessins

L'invention sera bien comprise à la lecture de la description suivante d'exemples de réalisation, en référence aux dessins annexés dans lesquels :
[Fig. 1] est un diagramme montrant la viabilité cellulaire de cellules neuronales HT22 traitées soit avec 50 µM de différentes molécules xanthophylles (rectangles blancs) pendant deux heures suivi d'une induction de mort cellulaire à l'aide de glutamate 10 mM, après incubation durant 24 heures à 37°C (rectangles noirs) ;
[Fig. 2] est un graphique présentant la viabilité cellulaire en pourcentages en fonction de la concentration en µM de diadinoxanthine seule (ligne en pointillés) et de diadinoxanthine en présence de glutamate (ligne pleine);
[Fig. 3] est un graphique présentant la viabilité cellulaire en pourcentages en fonction de la concentration en µM de violaxanthine seule (ligne en pointillés) et de violaxanthine en présence de glutamate (ligne pleine) ;
[Fig. 4] est un exemple comparatif présentant la viabilité cellulaire en pourcentages en fonction de la concentration en µM de diatoxanthine seule (ligne en pointillés) et de diatoxanthine en présence de glutamate (ligne pleine).

### EXEMPLES

Exemple 1 : des molécules pures (commercialisées par la Société SIGMA ALDRICH) de diadinoxanthine et de violaxanthine ainsi que d'autres molécules xanthophylles telles que la canthaxanthine, l'astaxanthine, la diatoxanthine, la fucoxanthine, le fucoxanthinol, la lutéine et la zéaxanthine ont été testées.

La violaxanthine peut être extraite de *Chlorella vulgaris* ou de *Tetraselmis suesica.* La diadinoxanthine peut être extraite des micro-algues *Odontella aurita* ou *Isochrysis galbana.*

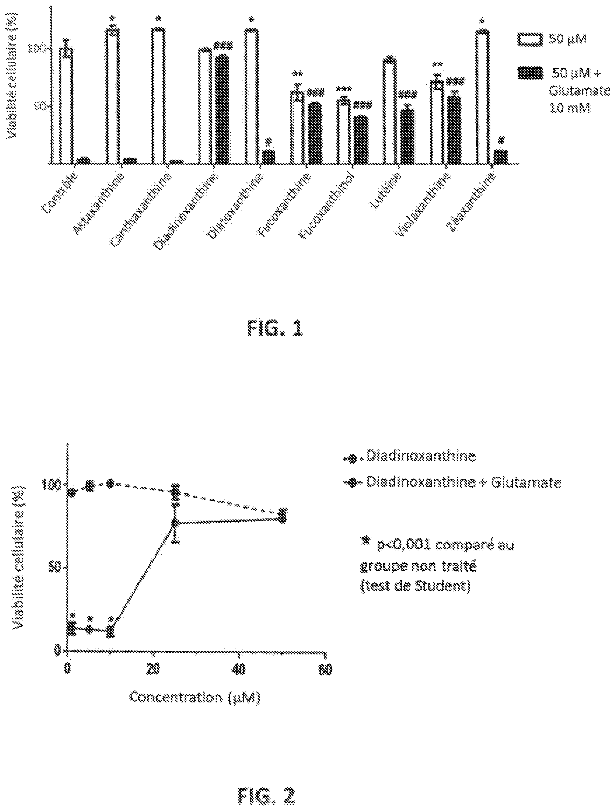

Les cellules neuronales HT22 (lignée neuronale d'hippocampe de souris) ont été traitées pendant deux heures par 50 µM de pigment suivi ou non de l'ajout de 10 mM de glutamate pendant 24 heures à 37°C. La viabilité cellulaire (en %) a été mesurée par un test MTS (au moyen d'un kit CellTiter 96^{®}Aqueous non-Radioactive Cell Proliferation Assay de la société Promega). Les valeurs présentées sur la figure 1 sont la moyenne ± écart type(n=3), ★ P<0,05 ; ★★ P<0,01 ; ★★★ P<0,001 par rapport au contrôle cellulaire non traité. # P<0,05 ; ### P<0,001 par rapport au contrôle cellulaire induit au glutamate.

Ces résultats montrent l'effet non toxique à 50µM de la plupart des pigments testés (rectangles blancs) et l'effet protecteur à plus de 50 % de la diadinoxanthine et de la violaxanthine contre la cytotoxicité du glutamate sur ces cellules neuronales HT22 (rectangles noirs). La morphologie des cellules est obtenue par INCUCYTE^{®}S3.

Nous voyons donc que les pigments de diadinoxanthine et violaxanthine ne sont pas toxiques et génèrent un effet protecteur sur la mort cellulaire des cellules neuronales HT22 induite par le glutamate à 10 mM (Figure 1).

Exemple 2 : suite à l'exemple 1 ayant donné des résultats intéressants pour la diadinoxanthine et la violaxanthine, des tests de dose-réponse entre 1 et 50 µM sur le même modèle cellulaire HT22 induit au glutamate (10 mM) ont été réalisés.

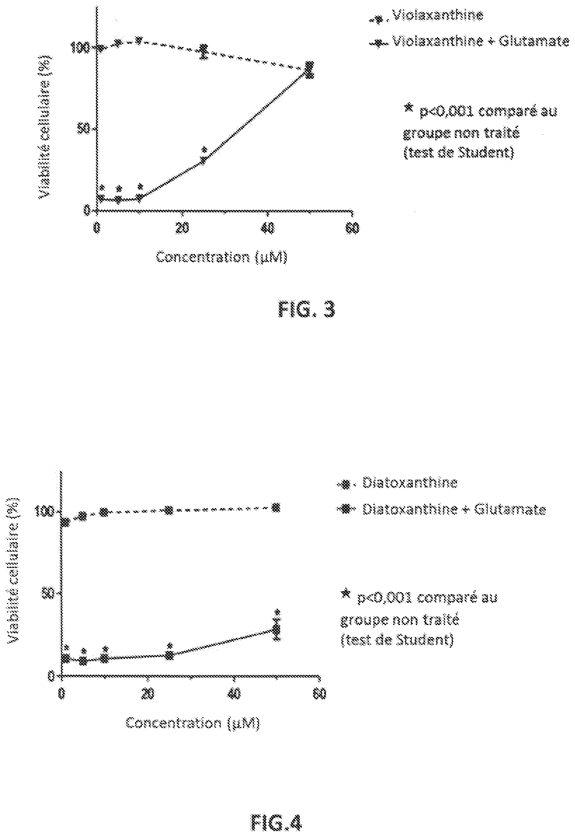

Les cellules HT22 ont été traitées durant 2 heures avec soit la diadinoxanthine (Figure 2) soit la violaxanthine (Figure 3) soit la diathoxanthine (essai comparatif présenté sur la figure 4) à différentes concentrations. Pour chacune des concentrations, la mort cellulaire a ensuite été induite par le glutamate (10 mM) durant 24 heures à 37°C. La viabilité cellulaire (en %) a été mesurée par un test au MTS. Les valeurs représentées sont la moyenne ± écart-type (n=3).

La CE50 (ou Concentration Efficace médiane) est la concentration à laquelle une molécule induit une réponse à mi-chemin entre la ligne de base et l'effet maximum après un certain temps d'exposition à celle-ci. Cela détermine son efficacité. Pour la diadinoxanthine la valeur de CE50 est de 22 µM et celle de la violaxanthine de 28 µM.

Ces résultats démontrent clairement que ces deux molécules protègent les neurones contre la mort cellulaire, cet effet surprenant étant supérieur à celui obtenu par les autres molécules xanthophylles déjà validées sur les animaux pour diminuer le risque de maladies neurodégénératives (publication de Cho *et al.,* citée plus haut).

## Revendications

1. Composition renfermant des pigments caroténoïdes de la famille des xanthophylles, **caractérisée en ce que** lesdits pigments de la famille des xanthophylles comprennent au moins l'une des molécules de formule (I) ou (II) suivantes : 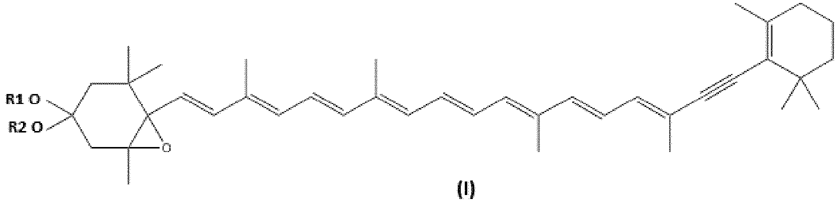 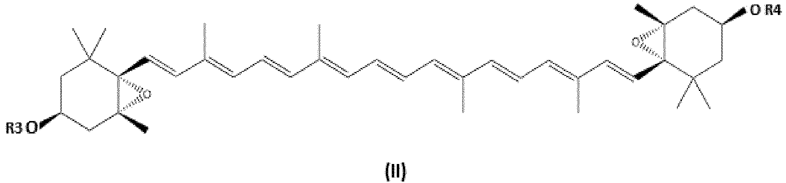 dans lesquelles les radicaux R1, R2, R3 et R4, identiques ou différents, sont choisis parmi l'hydrogène, un radical hydrocarboné linéaire ou ramifié, saturé ou insaturé, tel qu'un radical alkyle en C₁ à C₃₆, ou les groupements -OR1, -OR2, -OR3 ou -OR4 sont des esters d'acides gras en C₄ à C₃₆, de préférence en C₄ à C₂₈, pour une utilisation dans la prévention ou le traitement du déclin cognitif ou de maladies neurodégénératives.

2. Composition pour son utilisation selon la revendication 1, **caractérisée en ce que** la molécule de la famille des xanthophylles de formule (I) est la diadinoxanthine, ou un ester d'acide gras en C₄ à C₃₆ de la diadinoxanthine.

3. Composition pour son utilisation selon la revendication 1 , **caractérisée en ce que** la molécule de la famille des xanthophylles de formule (II) est la violaxanthine, ou un ester d'acide gras en C₄ à C₃₆ de la violaxanthine.

4. Composition pour son utilisation selon l'une quelconque des revendications précédentes **caractérisée en ce que** les molécules xanthophylles sont extraites de micro-algues marines ou d'eau douce, de macro-algues marines, de cyanobactéries ou de bactéries.

5. Composition pour son utilisation selon l'une quelconque des revendications précédentes **caractérisée en ce que** les molécules xanthophylles de formule (I) sont extraites d'algues euglénophytes de l'embranchement Euglenozoa, de diatomées de l'embranchement Bacillariophyta, de dinoflagellés de la classe Dinophyceae, d'Haptophytes, de Pelagophytes, de Phaetothamniophytes, de Dictyochophytes ou d'Ochrophytes.

6. Composition pour son utilisation selon l'une quelconque des revendications précédentes **caractérisée en ce que** les molécules xanthophylles de formule (II) sont extraites d'algues des embranchements des Chrysophytes, des Eustigmatophytes, des Synurophytes, des Mesostigmatophytes, des Chlorophytes, des Prasinophytes, des Chlorarachniophytes, des Pinguiophytes, des Raphidophytes, ou extraites des dinoflagellés, ou de macro-algues.

7. Composition pour son utilisation selon l'une des revendications précédentes, **caractérisée en ce que** les molécules xanthophylles de formule (I) ou (II) sont obtenues à partir de micro-algues, au moyen d'un procédé comprenant les étapes successives suivantes :
i) broyage des micro-algues et dispersion dans de l'éthanol dans un broyeur-mélangeur à billes permettant l'extraction des xanthophylles,
ii) après élimination desdites billes, centrifugation pour recueillir le surnageant,
iii) filtration du surnageant sur un filtre de 0,2 µm,
iv) suivie immédiatement d'une séparation et purification des pigments par chromatographie de partage centrifuge ou par chromatographie liquide haute performance et détection UV des fractions éluées,
v) séchage et conditionnement.

8. Composition pour son utilisation selon l'une quelconque des revendications précédentes, **caractérisée en ce qu'**elle est utilisée en tant que substance active dans un médicament.

9. Composition pour son utilisation selon l'une quelconque des revendications précédentes pour une utilisation dans la prévention ou le traitement de la maladie d'Alzheimer, la maladie de Parkinson, l'épilepsie, les déficiences intellectuelles/autisme, l'ataxie spinocérébelleuse, la sclérose en plaques, les démences à corps de Lewy, la maladie d'Alexander, la maladie d'Alpers, l'atrophie multi-systématisée, l'atrophie cortico postérieure (syndrome de Benson), la dégénérescence cortico-basale, la paralysie supranucléaire progressive, la maladie de Pick, la myofasciite à macrophages, la maladie de Huntington, la sclérose latérale amyotrophique, la maladie de Creutzfeldt-Jakob, ou la neurodégénération glaucomateuse telle que le glaucome primaire à angle ouvert.

10. Composition pour son utilisation selon l'une quelconque des revendications précédentes **caractérisé en ce qu'**elle comprend au moins un élément supplémentaire choisi parmi :
- les vitamines suivantes : E, B3, B5, B6, H, B9 et B12 ;
- les oligo-éléments et minéraux suivants : magnésium, iode et sélénium ;
- les composés suivants : acide eicosapentaénoïque (EPA), acide docosahexaénoïque (DHA), et lécithine de tournesol ;
- des protéines végétales ;
- un extrait de myrtille ou d'une autre plante ;
- les caroténoïdes suivants : astaxanthine, fucoxanthine, lutéine, zéaxanthine, alpha-carotène, beta-carotène, crocétine, crocine, beta-cryptoxanthine et lycopène ;
- des excipients, émulsifiants, agents de charge, épaississants, antiagglomérants, stabilisants, acidifiants et arômes.

## Patentansprüche

1. Zusammensetzung, die Carotinoidpigmente aus der Familie der Xanthophylle enthält, **dadurch gekennzeichnet, dass** die genannten Pigmente aus der Familie der Xanthophylle mindestens eines der Moleküle der folgenden Formel (I) oder (II) umfassen: 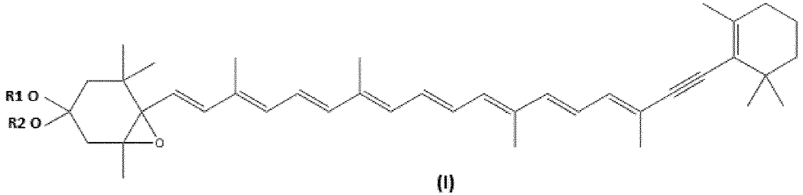 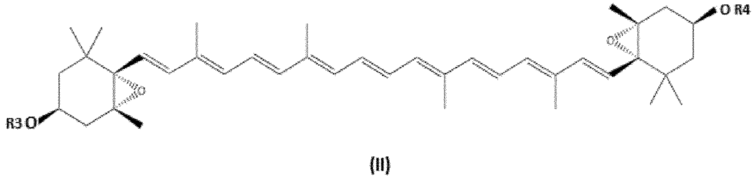 in denen die Radikale R1, R2, R3 und R4, die identisch oder verschieden sind, ausgewählt sind aus Wasserstoff, einem linearen oder verzweigten, gesättigten oder ungesättigten Kohlenwasserstoffradikal, wie einem C₁ bis C₃₆ Alkylradikal, oder die Gruppen -OR1, -OR2, -OR3 oder -OR4 Ester von C₄ bis C₃₆ Fettsäuren, vorzugsweise C₄ bis C₂₈, zur Verwendung bei der Prävention oder Behandlung von kognitivem Abbau oder neurodegenerativen Erkrankungen sind.

2. Zusammensetzung zur Verwendung nach Anspruch 1, **dadurch gekennzeichnet, dass** das Molekül aus der Familie der Xanthophylle von Formel (I) Diadinoxanthin oder ein C₄ bis C₃₆ Fettsäureester von Diadinoxanthin ist.

3. Zusammensetzung zur Verwendung nach Anspruch 1, **dadurch gekennzeichnet, dass** das Molekül aus der Familie der Xanthophylle von Formel (II) Violaxanthin oder ein C₄ bis C₃₆ Fettsäureester von Violaxanthin ist.

4. Zusammensetzung zur Verwendung nach einem der vorherigen Ansprüche, **dadurch gekennzeichnet, dass** die Xanthophyllmoleküle aus Meeres- oder Süßwassermikroalgen, Meeresmakroalgen, Cyanobakterien oder Bakterien extrahiert sind.

5. Zusammensetzung zur Verwendung nach einem der vorherigen Ansprüche, **dadurch gekennzeichnet, dass** die Xanthophyllmoleküle von Formel (I) aus euglenophytischen Algen des Stammes Euglenozoa, aus Kieselalgen des Stammes Bacillariophyta, aus Dinoflagellaten der Klasse Dinophyceae, aus Haptophyten, Pelagophyten, Phaetothamniophyten, Dictyochophyten oder Ochrophyten extrahiert sind.

6. Zusammensetzung zur Verwendung nach einem der vorherigen Ansprüche, **dadurch gekennzeichnet, dass** die Xanthophyllmoleküle von Formel (II) aus Algen der Stämme Chrysophyten, Eustigmatophyten, Synurophyten, Mesostigmatophyten, Chlorophyten, Prasinophyten, Chlorarachniophyten, Pinguiophyten, Raphidophyten extrahiert oder aus Dinoflagellaten oder aus Makroalgen extrahiert sind.

7. Zusammensetzung zur Verwendung nach einem der vorherigen Ansprüche, **dadurch gekennzeichnet, dass** die Xanthophyllmoleküle von Formel (I) oder (II) aus Mikroalgen mittels eines Verfahrens gewonnen werden, das die folgenden aufeinanderfolgenden Schritte beinhaltet:
i) Zerkleinern der Mikroalgen und Dispergieren in Ethanol in einem Kugelmühlenmischer, der die Extraktion von Xanthophyllen ermöglicht,
ii) Zentrifugieren, nach dem Entfernen der genannten Kügelchen, zum Sammeln des Überstands,
iii) Filtrieren des Überstands durch einen 0,2 µm Filter,
iv) unmittelbar gefolgt von einer Trennung und Reinigung der Pigmente durch Zentrifugalteilungschromatographie oder durch Hochleistungsflüssigkeitschromatographie und UV-Nachweis der eluierten Fraktionen,
v) Trocknen und Verpacken.

8. Zusammensetzung zur Verwendung nach einem der vorherigen Ansprüche, **dadurch gekennzeichnet, dass** sie als Wirkstoff in einem Arzneimittel verwendet wird.

9. Zusammensetzung zur Verwendung nach einem der vorherigen Ansprüche zur Verwendung bei der Prävention oder Behandlung von Alzheimer-Krankheit, Parkinson-Krankheit, Epilepsie, geistiger Behinderung/Autismus, spinozerebellärer Ataxie, Multipler Sklerose, Lewy-Körperchen-Demenz, Alexander-Krankheit, Alpers-Krankheit, Multi-Systematrophie, kortiko-posteriorer Atrophie (Benson-Syndrom), kortiko-basaler Degeneration, progressiver supranukleärer Lähmung, Pick-Krankheit, Makrophagenmyofasziitis, Huntington-Krankheit, amyotropher Lateralsklerose, Creutzfeldt-Jakob-Krankheit oder glaukomatöser Neurodegeneration wie das primäre Offenwinkelglaukom.

10. Zusammensetzung zur Verwendung nach einem der vorherigen Ansprüche, **dadurch gekennzeichnet, dass** sie mindestens ein zusätzliches Element enthält, ausgewählt aus:
- den folgenden Vitaminen: E, B3, B5, B6, H, B9 und B12;
- den folgenden Spurenelementen und Mineralien: Magnesium, Jod und Selen;
- den folgenden Verbindungen: Eicosapentaensäure (EPA), Docosahexaensäure (DHA) und Sonnenblumenlecithin;
- pflanzlichen Proteinen;
- einem Extrakt aus Heidelbeeren oder einer anderen Pflanze;
- den folgenden Carotinoiden: Astaxanthin, Fucoxanthin, Lutein, Zeaxanthin, Alpha-Carotin, Beta-Carotin, Crocetin, Crocin, Beta-Cryptoxanthin und Lycopin;
- Hilfsstoffen, Emulgatoren, Füllstoffen, Verdickungsmitteln, Antiklumpmitteln, Stabilisatoren, Säuerungsmitteln und Aromastoffen.

## Claims

1. Composition containing carotenoid pigments of the xanthophyll family, **characterized in that** said pigments of the xanthophyll family comprise at least one of the molecules of the following formula (I) or (II): 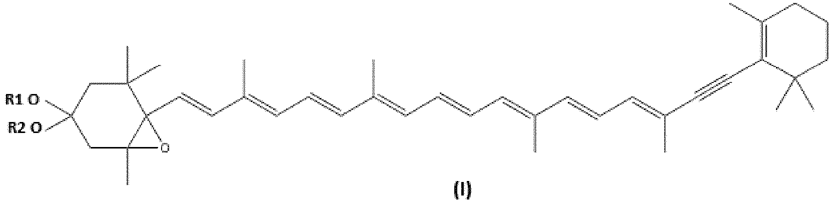 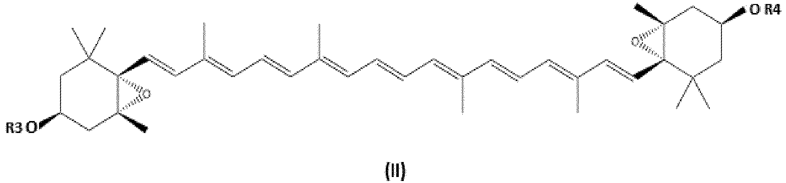 in which the R1, R2, R3 and R4 radicals, identical or different, are selected from hydrogen, a saturated or unsaturated, linear or branched hydrocarbon radical, such as a C₁ to C₃₆ alkyl radical, or the groups -OR1, -OR2, -OR3 or - OR4 are C₄ to C₃₆, preferably C₄ to C₂₈, fatty acid esters, for use in the prevention or treatment of cognitive decline or neurodegenerative diseases.

2. Composition for its use according to claim 1, **characterized in that** the molecule of the xanthophyll family of formula (I) is diadinoxanthin, or a C₄ to C₃₆ fatty acid ester of diadinoxanthin.

3. Composition for its use according to one of claims 1 or 2, **characterized in that** the molecule of the xanthophyll family of formula (II) is violaxanthin, or a C₄ to C₃₆ fatty acid ester of violaxanthin.

4. Composition for its use according to any one of the preceding claims, **characterized in that** the xanthophyll molecules are extracted from marine or freshwater microalgae, marine macroalgae, cyanobacteria or bacteria.

5. Composition for its use according to any one of the preceding claims, **characterized in that** the xanthophyll molecules of formula (I) are extracted from euglenophyte algae of the Euglenozoa phylum, diatoms of the Bacillariophyta phylum, dinoflagellates of the Dinophyceae class, Haptophytes, Pelagophytes, Phaetothamniophytes, Dictyochophytes or Ochrophytes.

6. Composition for its use according to any one of the preceding claims, **characterized in that** the xanthophyll molecules of formula (II) are extracted from algae of the phyla Chrysophytes, Eustigmatophytes, Synurophytes, Mesostigmatophytes, Chlorophytes, Prasinophytes, Chlorarachniophytes, Pinguiophytes, Raphidophytes, or extracted from the dinoflagellates, or from macro-algae.

7. Composition for its use according to any one of the preceding claims, **characterized in that** the xanthophyll molecules of formula (I) or (II) are obtained from microalgae, by means of a method comprising the following successive steps:
i) grinding the microalgae and dispersion in ethanol in a ball mixer-grinder allowing the extraction of the xanthophylls,
ii) after elimination of said balls, centrifugation for recovering the supernatant,
iii) filtering the supernatant over a 0.2 µm filter,
iv) followed immediately by a separation and purification of the pigments by centrifugal partition chromatography or by high-performance liquid chromatography and UV detection of the eluted fractions,
v) drying and packaging.

8. Composition for its use according to any one of the preceding claims, **characterized in that** it is used as an active substance in a medicament.

9. Composition for its use according to any one of the preceding claims, for a use in the prevention or the treatment of Alzheimer's disease, Parkinson's disease, epilepsy, learning disabilities/autism, spinocerebellar ataxia, multiple sclerosis, Lewy body dementia, Alexander disease, Alpers disease, multiple system atrophy, posterior cortical atrophy (Benson's syndrome), cortico-basal degeneration, progressive supranuclear palsy, Pick's disease, macrophagic myofasciitis, Huntington's disease, amyotrophic lateral sclerosis, Creutzfeldt-Jakob disease, or glaucomatous neurodegeneration such as primary open angle glaucoma.

10. Composition for its use according to any one of the preceding claims, **characterized in that** it comprises at least one additional element selected from:
- the following vitamins: E, B3, B5, B6, H, B9 and B12;
- the following trace elements and minerals: magnesium, iodine and selenium;
- the following compounds: eicosapentaenoic acid (EPA), docosahexaenoic acid (DHA), and sunflower lecithin;
- plant proteins;
- an extract of blueberry or another plant;
- the following carotenoids: astaxanthin, fucoxanthin, lutein, zeaxanthin, alpha-carotene, beta-carotene, crocetin, crocin, beta-cryptoxanthin and lycopene;
- excipients, emulsifiers, bulking agents, thickeners, anti-caking agents, stabilizers, acidifiers and flavourings.
